# EUROPEAN PATENT SPECIFICATION

(11) **EP 2 575 807 B1**
(45) Date of publication and mention of the grant of the patent: **19.02.2014**
(21) Application number: 10729933.1
(22) Date of filing: 28.05.2010
(51) Int. Cl.: A61K 31/40, A61K 41/00, A61P 25/24, A61P 25/20, A61P 25/22

(54) **FORMULATION BASED ON SULPIRIDE AND A SYNERGISTIC HOMEOPATHIC COMPOSITION FOR TREATMENT OF DEPRESSIVE SYNDROME**
FORMULIERUNG ENTHALTEND SULPIRID UND EINE SYNERGISTISCH WIRKENDE HOMÖOPATHISCHE ZUSAMMENSETZUNG ZUR BEHANDLUNG VON DEPRESSIONEN
FORMULATION COMPRENANT DU SULPIRIDE ET UNE COMPOSITION HOMEOPATHIQUE SYNERGISTIQUE POUR LE TRAITEMENT DE LA DEPRESSION

(43) Date of publication of application: 10.04.2013
(73) Proprietor: Novak, Maja, 10000 Zagreb (HR)
(72) Inventor: Novak, Maja, 10000 Zagreb (HR)
(74) Representative: Bihar, Zeljko
(86) International application number: PCT/HR2010/000015
(87) International publication number: WO 2011/148203

(56) References cited:
- BRUYNOOGHE F ET AL: "Treatment of reactive depression with sulpiride. Double-blind study with sulpiride and amitriptyline in an interindividual comparison /// Behandlung reaktiver Depressionen mit Sulprid. Doppelblindstudie mit Sulpirid und Amitritylin im interindividuellen Vergleich" FORTSCHRITTE DER MEDIZIN, URBAN UND VOGEL, MUENCHEN, DE, vol. 110, no. 27, 30 September 1992 (1992-09-30), pages 498-502, XP009097518 ISSN: 0015-8178
- DATABASE WPI Week 200828 Thomson Scientific, London, GB; AN 2008-D86806 XP002600542 & CN 101 081 272 A (ZHANG B) 5 December 2007 (2007-12-05)
- Katz T: "The feasibility of a randomised, placebo-controlled clinical trial of homeopathic treatment of depression in general practice" Homeopathy vol. 94, 2005, pages 145-152, XP002600543 DOI: doi:10.1016/j.homp.2005.04.002 [retrieved on 2010-09-14]

## Description

### FIELD OF THE INVENTION

The present invention relates to a formulation based on sulpiride and a synergistic homeopathic composition that exhibits a profound antidepressant activity.

### SUMMARY OF THE INVENTION

This invention discloses an improved pharmaceutical product which is used as antidepressant, based on the formulation comprising:
(i) sulpiride (1); 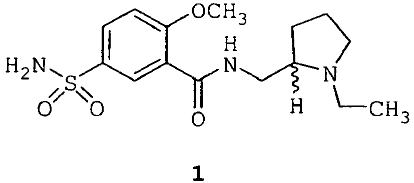
(ii) a homeopathic composition consisting of the Krebs cycle involving compounds:
   (a) intermediates: citric acid (D8), *cis*-aconitic acid (D8), α-ketoglutaric acid (D8), succinic acid (D10), fumaric acid (D8), DL-malic acid (D8), sodium diethyloxaloacetate (D6), sodium pyruvate (D8), barium oxalosuccinate (D10);
   (b) vitamins, nucleosides, and their biosynthesis intermediates: coenzym A (D8), nicotinamide adenine dinucleotide (NAD; D8), adenosine triphosphate (ATP; D10), ascorbic acid (D6), thiamine hydrochloride (D6), sodium riboflavin phosphate (D6), pyridoxine hydrochloride (D6), nicotinamide (D6), cysteine (D6), DL-α-lipoic acid (D6);
   (c) minerals: magnesium orotate (D6), cerium oxalate (D8), manganese phosphate (D6);
   (d) herbal extracts of the Small Pasque flower (*Pulsatilla pratensis L*.; D6), and the Red beet (*Beta vulgaris ssp. vulgaris var. conditiva,* D4); and
   (e) miscellaneous ingredients: sulphur (D10), and liver of sulphur (D10);
   all of them in equal relative amounts; and of
(iii)one or more excipients which yield in desired final dosage form: injection, tablets, capsules, oral solution, and syrup.

The homeopathic composition described under point (ii) can be considered as modified Krebs cycle solution (or homeopathic modified Krebs solution).

The above cited formulation exhibits a profound antidepressant activity due to synergistic action of homeopathic composition of Krebs cycle-involving components and herbal extracts, providing:
(i) potent antidepressant action at dosages of sulpiride of several times lower than is usual according to conventional clinical procedures;
(ii) absence of side-effects of sulpiride due to significantly reduced dosages; as well as
(iii) substantial anxiolitic and hypnotic activities.

### PRIOR ART

Beside cardiovascular and cancer diseases, depression is one of the most important disorders that affects the modern people. The depressive disorder is a serious illness that affects energy, sleep, appetite, libido, and the ability to function (expecially major depression), and has to be treated with various drugs usually called antidepressants. The latter involve several classes of compounds:
(i) tricyclic/polycyclic antidepressants (e.g. amitriptyline);
(ii) selective serotonin reuptake inhibitors (e.g. fluoxetine);
(iii)monoamine oxidase inhibitors;
(iv) lithium salts (e.g. lithium carbonate; mainly for treatment of mania); as well as
(v) certain atypical antipsychotics (e.g. sulpiride).

Classical therapies of depression often cause several unwanted side-effects like nausea, anxiety, insomnia, sexual dysfunction, anorexia, weight loss, and tremors [M. J. Mycek, R. A. Harvey, P. C. Champe: Pharmacology, 2nd Edition (1997) Lippincott-Raven Pub., Philadelphia, USA].

Sulpiride (**1**) is a well-known active pharmaceutical ingredient (API) from the class of dopamine D₂ and D₃ receptor antagonists with antipsychotic and antidepressant activities.

Main pharmacological use of sulpiride involves treatment of psychoses such as schizophrenia at daily dosages of 400-1.600 mg [G. Alfredsson, F. A. Wiesel: Relationships between clinical effects and monoamine metabolites and amino acids in sulpiride-treated schizophrenic patients, Psychopharmacology (Berl.) 101 (1990) 324-331; G. Alfredsson, L. Bjerkenstedt, G. Edman, C. Harnryd, G. Oxenstierna, G. Sedvall, F. A. Wiesel: Relationships between drug concentrations in serum and CSF, clinical effects and monoaminergic variables in schizophrenic patients treated with sulpiride or chlorpromazine, Acta Psychiatr. Scand. Suppl. 311 (1984) 49-74].

The use of sulpiride as antidepressant has been a subject of controversy. However lower daily dosage (100-200 mg) of sulpiride is generally considered as second-line antidepressant therapy [O. Benkert, F. Holsboer: Effect of sulpiride in endogenous depression, Acta Psychiatr. Scand. Suppl. 311 (1984) 43-48; W. Maier, O. Benkert: Treatment of chronic depression with sulpiride: evidence of efficacy in placebo-controlled single case studies, Psychopharmacology (Berl.) 11 (1994) 495-501; J. L. Lestynek: [Sulpiride and depression; in French] Sem. Hop. 59 (1983) 2354-2357].

Due to relatively low oral bioavailability of only 25-35%, sulpiride is usually administered either in 2-3 divided oral doses, or by intravenous injection.

Table 1 shows effects of sulpiride in combinations with various APIs:

**Table 1**

| | **Sulpiride combined with** | **Publication No. Applicant** | **Pharmacological activity** |
|---|---|---|---|
| 1. | cannabinoids | GB2456183 | antipsychotic |
| | | GW PHARMA LTD, ... | |
| 2. | sertraline | CN101138562 | antidepressant |
| | | YANFANG CHEN | |
| 3. | venlafaxine | CN1931153 | antidepressant |
| | | CHEN YANFANG | |
| 4. | oxybutynin and tolterodine | US2008207737 | treatment of hyperhidrosis |
| | | ZINGER MENNI | |
| 5. | trazodone | RU2334509 | treatment of eczema |
| | | GOU VPO VOENNO MED ... | |
| 6. | chlorimipramine, sodium valproate, borneol and certain herbal extract | CN101081272 | antipsychotic |
| | | BAOSHAN ZHANG | |
| 7. | penfluridol, clonazepam | CN1602877 | treatment of somatization disorder |
| | | XUE ZHONGJIE | |
| 8. | 5HT2C antagonists | KR20000029564 | antipsychotic |
| | | SMITHKLINE BEECHAM PLC | |
| 9. | cocaine | KR20000023672 | antiglaucoma |
| | | LIN TONGHO | |
| 10. | amitriptyline, nimodipine, borneol and certain chinese herbal extracts | CN1219416 | treatment of negative depressive psychosis |
| | | CHEN JINYAN | |
| 11. | ethaperazin, nimodipine, borneol and certain chinese herbal extracts | CN1219415 | treatment of chronic recessive psychosis |
| | | CHEN JINYAN | |
| 12. | amitriptiline, borneol and certain chinese herbal extracts | CN1149475 | treatment of negative depressive psychosis |
| | | LIN ZUXIAN | |
| 13. | composition of ten chinese healing herbs and penfluride | CN1171264 | treatment of psychoses such as manic depression and schizophrenia |
| | | SONG YANG | |
| 14. | clozapine | JP10175865 | antiglaucoma |
| | | RIN TOWA | |
| 15. | clozapine | US5744468 | antiglaucoma |
| | | LIN TONG HO | |
| 16. | clozapine | AU7024596 | antiglaucoma |
| | | TONG HO LIN | |
| 17. | perphenazine, trihexyphenidyl hydrochloride and extracts of certain chinese healing herbs | CN1149476 | treatment of chronic retraction psychosis |
| | | LIN ZUXIAN | |
| 18. | maprotiline, mianserine | WO940213 | treatment of serotonin depletion illness |
| | | IVY MARY ELIZABETH | |
| 19. | diazepam | FR2592793 | treatment of stress-linked digestive disorders |
| | | PF MEDICAMENT | |
| 20. | synthetic progestagens | ES8706441 | increasing level of prolactine in blood |
| | | ILE DE FRANCE | |
| 21. | synthetic progestagens | CA1256802 | increasing level of prolactine in blood |
| | | ILE DE FRANCE | |
| 22. | domperidone, ergocristine derivatives, 2-mercaptoethanol | CA1256379 | stimulation of hair growth |
| | | SANDOZ LTD | |
| 23. | GABA mimetic | FR2453643 | treatment of psychosis like schizophrenia |
| | | SYNTHELABO | |
| 24. | bromazepam | GB1393226 | increasing efficacy in treatment of psychoses like schizophrenia |
| | | HOFFMANN LA ROCHE | |

Although certain homeopathic drugs have been widely employed in the treatment of depression [K. Pilkington, G. Kirkwood, H. Rampes, P. Fisher, J. Richardson: Homeopathy for depression: a systematic review of the research evidence, Homeopathy 94 (2005) 153-163; J. R. Davidson, R. M. Morrison, J. Shore, R. T. Davidson, G. Bedayn: Homeopathic treatment of depression and anxiety, Altern. Ther. Health Med. 3 (1997) 46-49; L. Makich, R. Hussain, J. H. Humphries: Management of depression by homeopathic practitioners in Sydney, Australia, Complement Ther. Med. 15 (2007) 199-206], the modified Krebs solution, like herein-mentioned, has not been recognized as sole homeopathic therapy, but has been used in combination with other homeopathic remedies. Also, so far, it has not been recognized as component in combined homeopathic-allopathic therapy.

German based company produces and sales such homeopathic modified Krebs solution for the indication: "stimulation of blocked enzymatic systems in degenerative diseases, as well as in defective enzymatic functions", which gives a property of unspecific metabolic activator according to the opinion of the manufacturer [A. Bier: Ordinatio Antihomotoxica et Materia Medica, Heel GmbH, Baden-Baden, Germany (1991)].

Any report of combined use of any either allopathic or homeopathic modified Krebs solution and any atypical antipsychotic like sulpiride for treatment of depression and/or anxiety has not be found in previously cited art.

Management of depression has been solved by the present invention on a new, unexpected and effective manner as will be disclosed bellow.

### DETAILED DESCRIPTION

### Pharmacological activity of the formulation of the invention

The present invention discloses an improved pharmaceutical product which is used as antidepressant, based on the formulation consisting of:
(i) sulpiride (**1**) or (*R,S*)-(±)-5-(aminosulfonyl)-*N*-[(1-ethylpyrrolidin-2-yl)methyl]-2-methoxybenzamide; 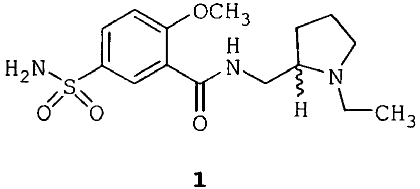
(ii) homeopathic composition comprising the Krebs cycle involving compounds:
   (a) intermediates: citric acid (D8), *cis*-aconitic acid (D8), α-ketoglutaric acid (D8), succinic acid (D10), fumaric acid (D8), DL-malic acid (D8), sodium diethyloxaloacetate (D6), sodium pyruvate (D8), barium oxalosuccinate (D10);
   (b) vitamins, nucleosides, and their biosynthesis intermediates: coenzym A (D8), nicotinamide adenine dinucleotide (NAD; D8), adenosine triphosphate (ATP; D10), ascorbic acid (D6), thiamine hydrochloride (D6), sodium riboflavin phosphate (D6), pyridoxine hydrochloride (D6), nicotinamide (D6), cysteine (D6), DL-α-lipoic acid (D6);
   (c) minerals: magnesium orotate (D6), cerium oxalate (D8), manganese phosphate (D6);
   (d) herbal extracts of the Small Pasque flower (*Pulsatilla pratensis L*.; D6), and the Red beet (*Beta vulgaris ssp. vulgaris var. conditiva,* D4); as well as (e) miscellaneous ingredients: sulphur (D10), and liver of sulphur (D10);
   all of them in equal relative amounts; and of
(iii) one or more excipients which yield in desired final dosage form: injection, tablets, capsules, oral solution, and syrup.

The letter "D" in each bracket stands for decimal dilution, whereas the following number represents the number of repeated (decimal) dilution procedures according to basic homeopathy principle [O. Weingarther: The nature of the active ingredient in ultra molecular dilutions, Homeopathy 96 (2007) 220-226]. In this manner, the remark "D6" means that corresponding solution of a given active homeopathic ingredient (drug) is obtained by dilution of starting (mother solution; according to German Book of homeopathic drugs; HAB = Homöopathische Arzneimittel Buch) solution by six (6) repeated decimal dilutions in a pre-defined diluent (usually in purified water or aqueous ethanol), e.g. 1 mL of mother solution (defined by the HAB) is diluted with 9 mL of diluent furnishing D1; this D1 solution (1 mL) is subsequently diluted with 9 mL of diluent giving D2 solution, etc.

The pharmacological activity of the formulation according to present invention was studied as follows:
The study of antidepressant activity was conducted on sixty-seven (67) women suffering of depressive syndrome. The patients were from 44 to 80 years old. They are divided onto two groups by randomization:
   (i) A control group (N = 32) received once-a-day combined (in the same syringe) dose of 20 mg of sulpiride (1; 0.4 mL) diluted with physiological solution (2.0 mL) by subcutaneous injection during three months (90 days).
      This dosage is considered as placebo since it is several times (5-7.5x) lower than a minimal known therapeutic dosage of sulpiride (100-150 mg) [W. Maier, O. Benkert: Treatment of chronic depression with sulpiride: evidence of efficacy in placebo-controlled single case studies, Psychopharmacol. (Berl.) 115 (1994) 495-501; O. Benkert, H. Hippius: Psychiatrische Pharmakotherapie, 5 Auflage, Springer Verlag, Berlin (1992), in German]
   (ii) Experimental group (N = 35) received once-a-day the formulation from the present invention in the form of injection (2.4 mL; see Example 1) comprising 20 mg of sulpiride (**1**) and the homeopathic modified Krebs solution of the following composition:
      citric acid (D8; 20 mg), *cis*-aconitic acid (D8; 20 mg), α-ketoglutaric acid (D8; 20 mg), succinic acid (D10; 20 mg), fumaric acid (D8; 20 mg), DL-malic acid (D8; 20 mg), sodium diethyloxaloacetate (D6; 20 mg), sodium pyruvate (D8; 20 mg), barium oxalosuccinate (D10; 20 mg), coenzym A (D8; 20 mg), nicotinamide adenine dinucleotide (NAD; D8; 20 mg), adenosine triphosphate (ATP; D10; 20 mg), ascorbic acid (D6; 20 mg), thiamine hydrochloride (D6; 20 mg), sodium riboflavin phosphate (D6; 20 mg), pyridoxine hydrochloride (D6; 20 mg), nicotinamide (D6; 20 mg), cysteine (D6; 20 mg), DL-α-lipoic acid (D6; 20 mg), sulphur (D10; 20 mg), liver of sulphur (D10; 20 mg), magnesium orotate (D6; 20 mg), cerium oxalate (D8; 20 mg), manganese phosphate (D6; 20 mg), the Small Pasque flower extract (*Pulsatilla pratensis L.;* D6; 20 mg), the Red beet extract (*Beta vulgaris ssp. vulgaris var. Conditiva;* D4; 20 mg), and sodium chloride (21.4 mg; 0.89%) in purified water (to 100%; 1.88 mL) as excipients.

The duration of administering was three months (90 days; also subcutaneously).

The study was followed by the use of Hamilton rating scale for depression (HRSD), also known as HAM-D or HAMD test, which is generally accepted as "golden standard" for quantification of severity of depression symptoms like: low mood, anxiety, agitation, insomnia, weight loss [M. Hamilton: A rating scale for depression, J. Neurol. Neurosurg. Psychiatr. 23 (1960) 56-62; J. L. Hedlung, B. W. Viewig: The Hamilton rating scale for depression: a comprehensive review, J. Oper. Psychiatr. 10 (1979) 149-165].

Results are presented in Table 2. showing Hamilton rating scale for depression (HAMD) test results of controlled-study of the antidepressant activity of the composition from the invention in comparison to placebo. For the 17-item version of HAMD test, scores can range from 0 to 54. One interpretation suggests that scores between 0 and 6 indicate a normal person with regard to depression, scores between 7 and 17 indicate mild depression, scores between 18 and 24 indicate moderate depression, and scores over 24 indicate severe depression.

**Table 2**

| **No.** | **Group:** | **HAMD** |
|---|---|---|
| | **Before the Study:** | |
| 1 | Control group (N = 32) | 18.91 ± 9.19 |
| 2 | Experimental group (N = 35) | 21.34 ± 5.05 |

| | **After the Study:** | |
|---|---|---|
| 3 | Control group (N = 32) | 17.34 ± 8.77 |
| 4 | Experimental group (N = 35) | 8.83 ± 4.14 |

The above results were subjected to paired T-test. The pairs were chosen to reflect changes during the Study in Control group (rows 1 and 3) and Experimental group (rows 1 and 4). Paired T-test is usually chosen to establish the difference between groups, i.e. their mean values during the Study.

The results of the paired T-tests performed by Analyse-it® version 2.21, Analyse-it Software, are shown in Table 3:

**Table 3**

| **Paired T-test parameters** | **Control group** | **Experimental group** |
|---|---|---|
| Mean difference | 1.60 | 12.5 |
| 95% Confidence interval (95% CI) | 0.9-2.2 | 10.9-14.2 |
| Standard Error (SE) | 0.31 | 0.81 |
| t-statistic | 4.97 | 15.47 |
| Degrees of freedom (DF) | 31 | 34 |
| 2-tailed "p" value | <0.0001 | <0.0001 |

Therefore, the results from the HAMD test strongly suggest that the formulation of the invention exhibits a strong antidepressant activity (see the "mean difference" - Table 3). It is observed decreasing of the HAMD mean score for 12.5 points for the Experimental group.

The absence of such strong decreasing of the HAMD mean score in Control group leads to conclusion of strong synergistic effect between homeopathic part of the composition and the sulpiride itself on the treated patients. According to all available data from the literature, sulpiride does not show any antidepressant activity at such low dosage (i.e. 20 mg/day). Thanks to this synergistic action of homeopathic Krebs cycle compounds, the corresponding required uptake (20 mg/day) of sulpiride through the formulation is approx. 7.5x lower than is usual dosage level of conventional antidepressant treatment with the same API (150 mg/day) [W. Maier, O. Benkert: Treatment of chronic depression with sulpiride: evidence of efficacy in placebo-controlled single case studies, Psychopharmacology (Berl.) 11 (1994) 495-501].

Beside a profound antidepressant activity, the results from the Hamilton test (HAMD) strongly suggest that the disclosed formulation also exhibits anxiolytic and hypnotic effects.

Finally the formulation of the present invention allows effective antidepressant therapy without side-effects which are common at classical dosage rates of sulpiride administration such as: sedation, constipation, and dryness of mouth [T. Tsukamoto, M. Asakura, T. Tsuneizumi, Y. Satoh, T. Shinozuka, K. Hasegawa: Therapeutic effects and side-effects in patients with major depression treated with sulpiride once a day, Prog. Neuropsychopharmacol. Biol. Psychiatry 18 (1994) 615-618].

### Composition of the formulation according to the invention

The formulation of this invention is consisting of:
(i) sulpiride (**1**), from 0.1-25% w/w;
(ii) the homeopathic composition comprising the Krebs cycle involving compounds and herbal extracts:
   citric acid (D8), *cis*-aconitic acid (D8), α-ketoglutaric acid (D8), succinic acid (D10), fumaric acid (D8), DL-malic acid (D8), sodium diethyloxaloacetate (D6), sodium pyruvate (D8), barium oxalosuccinate (D10), coenzym A (D8), nicotinamide adenine dinucleotide (NAD; D8), adenosine triphosphate (ATP; D10), ascorbic acid (D6), thiamine hydrochloride (D6), sodium riboflavin phosphate (D6), pyridoxine hydrochloride (D6), nicotinamide (D6), cysteine (D6), DL-α-lipoic acid (D6), sulphur (D10), liver of sulphur (D10), magnesium orotate (D6), cerium oxalate (D8), manganese phosphate (D6), the Small Pasque flower extract (*Pulsatilla pratensis L.;* D6), and the Red beet extract (*Beta vulgaris ssp. vulgaris var. conditiva,* D4); all of them in equal relative amounts; from 10-75% w/w; and of
(iii)one or more excipients which yield in desired final dosage form: injection, tablets, capsules, oral solution, and syrup, to yield 100% w/w of said formulation.

The excipients are selected from the groups consisting of diluents, isotonic salts, emulsifiers (solubilizers), thickeners, fillers, binders, disintegrants, lubricants, preservatives, antioxidants, and stabilizers.

In liquid final dosage forms such as injections, the diluent is a liquid compound selected from the group consisting of purified water, glycerol, 1,2-propyleneglycol, liquid polyethyleneglycols, liquid polyglycerols, aqueous sorbitol, dimethylsulfoxide, or mixtures of these substances. Beside mentioned, oral solutions can contain as diluent also 1,3-propyleneglycol and 1,3-butanediol.

In liquid final dosage forms such as syrups, the diluent is selected from the group comprising purified water, glycerol, 1,2-propyleneglycol, honey, aqueous sorbitol, or mixtures of these substances.

In injections, isotonic salts that can be used in preparation of the formulation is selected from the group consisting of sodium chloride (NaCl), potassium chloride (KCl), magnesium chloride (MgCl₂) and its hydrates, calcium chloride (CaCl₂) and its hydrates, or mixtures of these substances.

In liquid final dosage forms, the emulsifier (or solubilizer) is selected from the group comprising: sodium laurylsulphate, sodium lauryl ethyleneglycolsulphate, sodium lauryl diethyleneglycolsulphate, potassium laurylsulphate, potassium lauryl ethyleneglycolsulphate, potassium lauryl diethyleneglycolsulphate; sodium or potassium cocoamphodipropionate; disodium or dipotassium cocoamphodiacetate; polyoxyethylene(10) laurylether, polyoxyethylene(23) laurylether, polyoxyethylene(10) stearylether; polyoxyethylene(23) stearylether; polyoxyethylene(10) oleylether; polyoxyethylene(23) oleylether, other ethoxylates of higher fatty alcohols with H.L.B. value ≥10; polyoxyethylene(10) laurate, polyoxyethylene(23) laurate, polyoxyethylene(10) stearate, polyoxyethylene(23) stearate, polyoxyethylene(10) oleate, polyoxyethylene(23) oleate, other ethoxylates of higher fatty acids with H.L.B. value ≥10; polyglyceryl derivatives of higher fatty alcohols with H.L.B. value ≥10; polyglyceryl derivatives of higher fatty acids with H.L.B. value ≥10; polyoxyethylene sorbitan monolaurate, polyoxyethylene sorbitan monostearate, polyoxyethylene sorbitan monooleate, other sorbitan derivatives with H.L.B. value ≥10; or mixtures of these substances.

Thickeners in the liquid final dosage forms of the formulation are selected from the group consisting of: polyacrylic acid, its co-polymers, or their sodium, or potassium salts; methylcellulose; sodium carboxymethylcellulose; 2-hydroxyethylcellulose; 2-hydroxypropylcellulose; starch; modified starches; polyglycerols; polyethyleneglycols; gelatin; pectin; agar agar; carrageenans; gum arabic; alginic acid; sodium alginate; or mixtures of these substances.

Filler is selected from the group consisting of microcrystalline cellulose, lactose monohydrate, calcium hydrogenphosphate, calcium sulfate dihydrate, calcium carbonate, colloidal silicic acid, sorbitol, inulin, starch, modified starches, dextrin, glucose, fructose, basic magnesium carbonate, calcium silicate, saccharose, or mixtures of these substances.

In solid dosage forms, binders are selected from the group comprising gelatin, lactose monohydrate, sorbitol, saccharose, xylitol, maltitol, starch, modified starches, methylcellulose, 2-hydroxyethylcellulose, 2-hydroxypropylcellulose, 2-hydroxypropyl methylcellulose, sodium carboxymethylcellulose, polyethyleneglycols, polyglycerols, polyvinylpyrrolidone, polyvinylpyrrolidone co-polymers, carrageenans, maltodextrin, or mixtures of these substances.

Disintegrants in solid dosage forms are selected from the group consisting of starch, modified starches, sodium starch glycolate, methylcellulose, sodium carboxymethylcellulose, 2-hydroxyethylcellulose, 2-hydroxypropylcellulose, 2-hydroxypropyl methylcellulose, polyvinylpyrrolidone, polyvinylpyrrolidone co-polymers, or mixtures of these substances.

Lubricants in solid dosage forms are selected from the group consisting of magnesium stearate, calcium stearate, zinc stearate, stearic acid, talc, silicon dioxide, or mixtures of these substances.

Preservatives are selected from the group comprising methyl 4-hydroxybenzoate, ethyl 4-hydroxybenzoate, propyl 4-hydroxybenzoate, butyl 4-hydroxybenzoate, sorbic acid, potassium sorbate, benzoic acid, sodium benzoate, 2-phenoxyethanol, 4-chloro-m-cresol, thymol, eugenol, or mixtures of these substances.

Antioxidants are selected from the group consisting of 2,6-di-*terc-*butyl-4-hydroxytoluene (BHT), *terc*-butylhydroxyanisole (BHA), tocopherol, tocopheryl acetate, ascorbic acid, or mixtures of these substances.

Stabilizers are selected from the group consisting of disodium ethylenediamine tetraacetate (Na₂EDTA·2H₂O), disodium N-(2-hydroxyethyl)ethylenediamine triacetate [Na₂H(HEDTA)], disodium diethylenetriamine pentaacetate [Na₂H₃(DTPA)], disodium citrate [Na₂C(OH) (COOH) (CH₂COO)₂], or mixtures of these substances.

### Preparation of the formulation of the present invention

The formulation of this invention involves the final dosage forms of injections, tablets, capsules, oral solutions, and syrups. The formulation is produced by common procedures known to those skilled in the art of pharmaceutical technology [J. Swarbrick (Ed.): Encyclopedia of Pharmaceutical Technology, 3rd Ed. (2007) Informa Healthcare Inc., USA].

Injections are produced by dissolution of homeopathic Krebs cycle components and herbal extracts in purified water, followed by addition of sulpiride (**1**) and sodium chloride (or other physiologically compatible inorganic salt up to isotonic level). Thus obtained solution is subjected to sterilization by microfiltration followed by filling into glass ampoules under aseptic conditions.

Oral solutions are obtained by dissolution of sulpiride in purified water or its mixtures with ethanol, 1,2-propyleneglycol and similar diluents (solvents) with or without addition of pharmaceutically acceptable acids such as sulfuric, hydrochloric, phosphoric, citric, malic, fumaric, tartaric, benzenesulfonic acid, etc. The latter facilitate dissolution of sulpiride in water by forming its salts which are smoothly soluble in water and water-based mixtures. Then the homeopathic modified Krebs solution according to the invention is added, and the product is homogenized by short mixing. Finally the oral solution can be stabilized by addition of preservatives like methyl- and propyl-4-hydroxybenzoate, antioxidant (e.g. BHT), and stabilizer (e.g. Na₂EDTA).

Syrup is produced by homogenization of sulpiride and homeopathic modified Krebs solution according to the invention in viscous aqueous solution of saccharose, honey, glucose, fructose, sorbitol, or their mixtures. In the case of the use of artificial sweeteners, intermediate solution is thickened by addition of edible thickeners like gelatin, pectin, starch, modified starches, sodium carboxymethylcellulose, or mixtures of these substances. Sweetener is selected from the group consisting of sodium saccharin, acesulfame potassium, sucralose, sodium or calcium cyclamate, xylitol, sorbitol, glycyrrhizin, extract of *Liquorice* root, or mixtures of these substances.

For production of solid dosage forms, tablets and capsules, a liquid homeopathic part of the formulation is converted into a powder by adsorption onto an inert inorganic carrier. The latter can be a filler or a mixture of filler and binder as defined above, e.g. microcrystalline cellulose, and/or lactose monohydrate. This operation is performed in a vacuum evaporator (at laboratory scale), or in a spray-drier (at industrial scale).

Thus produced powderous homeopathic part of the formulation (adsorbed on the filler) is homogenized with sulpiride, and subjected to either:
(i) direct compression yielding tablets; or
(ii) wet granulation, followed by compression of thus obtained granulate.

Capsules are produced by filling of gelatin or vegetable capsules either with a homogeneous mixture of powderous homeopathic part of the formulation with sulpiride, or with afore-mentioned granulate (produced by wet granulation technique).

There exist a number of other techniques that can be applied in the production of the final dosage forms of the formulation, but such alternatives would remain within the scope of this invention.

### EXAMPLES

### General remarks

Explanation of ingredients of homeopathic modified Krebs solution:
(i) citric acid, cis-aconitic acid, α-ketoglutaric acid, succinic acid, fumaric acid, malic acid are well-known intermediates in metabolic Krebs (or citric acid) cycle which are chemically stable as free acids, whereas sodium diethyloxaloacetate, barium oxalosuccinate, and sodium pyruvate were used as chemically stable forms (salts) of corresponding: pyruvic, oxaloacetic, and oxalosuccinic acids;
(ii) vitamins (usually co-enzymes), enzymes precursors, and Krebs cycle-involving nucleic bases employed: coenzym A, nicotinamide adenine dinucleotide (NAD), adenosine triphosphate (ATP), ascorbic acid, nicotinamide, cysteine, DL-α-lipoic acid, whereas thiamine and pyridoxine were used as commercially available hydrochloride salts, and riboflavin as sodium phosphate salt of good water solubility;
(iii) minerals: magnesium orotate [magnesium bis(2,6-dioxo-3H-pyrimidine-4-carboxylate); CAS No. [27067-77-2]; C₁₀H₆MgN₄O₈ Mᵣ= 334.48], 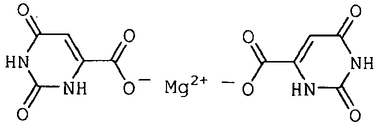 cerium(III) oxalate [Ce₂(C₂O₄)₃; CAS No. [139-42-4]; Mᵣ= 544.29], manganese(III) phosphate [Mn₃(PO₄)₂; Mᵣ= 354.76];
(iv) herbal extracts: Small Pasque flower extract (*Pulsatilla pratensis L*.), Red beet extract (*Beta vulgaris ssp. vulgaris var. Conditiva*) ; and
(v) miscellaneous homeopathic ingredients: sulphur (pharmaceutical-grade precipitated sulphur), and the liver of sulphur ["sulfurated potash"; in homeopthy known as "hepar sulfuris"; chiefly a mixture of potassium sulfide (K₂S), potassium polysulfides (K₂Sₓ; x= 2-6), potassium thiosulfate (K₂S₂O₅), and potassium sulfate (K₂SO₄); liver of sulfur is a commercially available product with wide technical and certain medicinal use - it is produced by heating of potassium carbonate (K₂CO₃) with excess of sulfur (S) in absence of air at 250 °C].

### Example 1

### Preparation of the formulation of the present invention in the form of injection

Composition (1000 mL of injection solution): (a) sulpiride (1; 8.33 g; 0.83% w/w), (b) homeopathic modified Krebs solution (216.67 g; 21.67% w/w), (c) sodium chloride (8.90 g; 0.89% w/w), (d) purified water (766.10 g; 76.61% w/w).

Procedure: Ingredient (b) was added into (d), and homogenized by stirring at room temperature (20-23 °C) for 5 minutes. Then (c) and (a) were added, and dissolved by mixing at room temperature during 15 minutes. Clear solution was subjected to microfiltration, followed by filling into transparent glass ampoules per 2.40 g (2.4 mL) under aseptic conditions.

These ampoules were employed during performing the study of antidepressant activity of the formulation of the invention. Each ampoule (2.4 mL) of thus prepared oral solution contains: 20 mg of sulpiride (**1**) and 260 mg of homeopathic modified Krebs solution (20 mg of each of its 26 components).

### Preparation of homeopathic modified Krebs solution (1000 g-scale):

Composition (1000 g-scale): (a) citric acid (D8; 38.46 g; 3.85% w/w), (b) *cis*-aconitic acid (D8; 38.46 g; 3.85% w/w), (c) α-ketoglutaric acid (D8; 38.46 g; 3.85% w/w), (d) succinic acid (D10; 38.46 g; 3.85% w/w), (e) fumaric acid (D8; 38.46 g; 3.85% w/w), (f) DL-malic acid (D8; 38.46 g; 3.85% w/w), (g) sodium diethyloxaloacetate (D6; 38.46 g; 3.85% w/w), (h) sodium pyruvate (D8; 38.46 g; 3.85% w/w), (i) barium oxalosuccinate (D10; 38.46 g; 3.85% w/w), (j) coenzym A (D8; 38.46 g; 3.85% w/w), (k) nicotinamide adenine dinucleotide (NAD; D8; 38.46 g; 3.85% w/w), (1) adenosine triphosphate (ATP; D10; 38.46 g; 3.85% w/w), (m) ascorbic acid (D6; 38.46 g; 3.85% w/w), (n) thiamine hydrochloride (D6; 38.46 g; 3.85% w/w), (o) sodium riboflavin phosphate (D6; 38.46 g; 3.85% w/w), (p) pyridoxine hydrochloride (D6; 38.46 g; 3.85% w/w), (q) nicotinamide (D6; 38.46 g; 3.85% w/w), (r) cysteine (D6; 38.46 g; 3.85% w/w), (s) DL-α-lipoic acid (D6; 38.46 g; 3.85% w/w), (t) sulphur (D10; 38.46 g; 3.85% w/w), (u) liver of sulphur (D10; 38.46 g; 3.85% w/w), (v) magnesium orotate (D6; 38.46 g; 3.85% w/w), (w) cerium oxalicum (D8; 38.46 g; 3.85% w/w), (x) manganese phosphate (D6; 38.46 g; 3.85% w/w), (y) Small Pasque flower extract (*Pulsatilla pratensis L.;* D6; 38.46 g; 3.85% w/w), (z) Red beet extract (*Beta vulgaris ssp. vulgaris var. Conditiva;* D4; 38.46 g; 3.85% w/w).

Corresponding homeopathic solutions of the components were prepared from mother solutions as described in German Book of Homeopathic drugs (HAB).

Procedure: Ingredients (a)-(z), each of 38.46 g were homogenized by stirring at room temperature for 15 minutes, filtered through microfilter, and filled into sterilized dark-glass bottle.

### Example 2

### Preparation of the formulation of the present invention in the form of oral solution

Composition (1000 g of oral solution): (a) sulpiride (**1**; 20.00 g; 2% w/w), (b) homeopathic modified Krebs solution (260.00 g; 26% w/w), (c) ethanol (300.00 g; 30% w/w), (d) purified water (420.00 g; 42% w/w).

Procedure: Ingredient (a) was added into previously prepared mixture of (c) and (d), and dissolved by stirring room temperature (20-23 °C) for 15 minutes. Then (b) was added, and homogenized by mixing at room temperature during 15 minutes. Clear colourless solution was subjected to microfiltration, followed by filling into a 100-mL dark glass bottles.

Homeopathic modified Krebs solution (ingredient b) was prepared according to the procedure described in Example 1.

Each mL of thus prepared oral solution contains: 20 mg of sulpiride (**1**) and 260 mg of homeopathic modified Krebs solution (10 mg of each of its 26 components).

### Example 3

### Preparation of the formulation of the present invention in the form of syrup

Composition (1000 g of syrup): (a) sulpiride (**1**; 4.00 g; 0.4% w/w), (b) homeopathic modified Krebs solution (104.00 g; 10.4% w/w), (c) saccharose (600.00 g; 60% w/w), (d) methyl 4-hydroxybenzoate (2.00 g; 0.2% w/w), (e) propyl 4-hydroxybenzoate (1.00 g; 0.1% w/w), (f) ethanol (96%; 10.00 g; 1% w/w), (g) BHT (0.01 g; 0.001% w/w), (h) Na₂EDTA (0.1 g; 0.01% w/w), (i) purified water (278.89 g; 27.9% w/w) .

Procedure: Ingredients (a), (g) and (h) were added into previously prepared mixture of (b) and (i), and dissolved by stirring room temperature (20-23°C) for 15 minutes. Then (c) was added, and dissolved by stirring at room temperature for 1 h. Preservatives (d) and (e) were dissolved in (f) by stirring for 5 minutes, and thus obtained clear solution was added to the rest of the syrup. The product was obtained in the form of colourless viscous liquid.

The syrup was filtered and filled into a 100-mL dark glass bottles. Homeopathic modified Krebs solution (ingredient b) was prepared according to the procedure described in Example 1.

Each 5 mL-dosing spoon of thus prepared syrup contains: 20 mg of sulpiride (1) and 520 mg of homeopathic modified Krebs solution (20 mg of each of its 26 components).

### Example 4

### Preparation of the formulation of the present invention in the form of tablets

Composition (1000 g of tablet mixture): (a) Microcrystalline cellulose (600.00 g; 60% w/w), (b) lactose monohydrate (305.89 g; 30.6% w/w), (c) sodium starch glycolate (25.00 g; 2.5% w/w), (d) polyvinylpyrrolidone (20.00 g; 2% w/w), (e) sulpiride (**1**; 40.00 g; 4% w/w), (f) homeopathic modified Krebs solution (300.00 g; corresponds to 150 mg per each 500 mg-tablet), (g) BHT (0.01 g; 0.001% w/w), (h) Na₂EDTA (0.1 g; 0.01% w/w), (i) magnesium stearate (9.00 g; 0.9% w/w).

Procedure: Ingredients (a), (g) and (h) were added into (f), and resulting thick paste was evaporated to dryness in a vacuum evaporator under deep vacuum at 40-45 °C. Thus obtained fine white off powder (600 g) was mixed with (b), (c), (d), and (e), and homogenized by mixing at room temperature (20-25 °C) for 30 minutes. Then (i) was added, and homogenization was continued for 15 minutes. Thus obtained homogeneous mixture was milled, and compressed into tablets yielding 2000 tablets (per 500 mg); Average tablet weight 494 mg.

Each tablet contains: 20 mg of sulpiride (**1**) and 150 mg of homeopathic modified Krebs solution (5.8 mg of each of its 26 components).

### Example 5

### Preparation of the formulation of the present invention in the form of capsules

Composition (1000 g of mixture for capsules): (a) Microcrystalline cellulose (945.50 g; 94-55% w/w), (b) sulpiride (**1**; 44.50 g; 4.45% w/w), (c) homeopathic modified Krebs solution (577.50 g; corresponds to 260 mg per each 450 mg net-weight capsule content), (d) magnesium stearate (10.00 g; 1% w/w).

Procedure: Ingredient (a) was added into (c), and resulting thick paste was evaporated to dryness in a vacuum evaporator under deep vacuum at 40-45 °C. Thus obtained fine white off powder (946 g) was mixed with (b) and (d), and homogenized by mixing at room temperature (20-25 °C) for 30 minutes. Thus obtained homogeneous mixture was filled into transparent hard gelatine capsules of size "0" (Lukaps; Ludbreg, Croatia), by using laboratory capsule filling machine. Yield approx. 2220 capsules with gross weight of 550 mg, and net weight of 450 mg.

Each capsule contains: 20 mg of sulpiride (**1**) and 260 mg of homeopathic modified Krebs solution (10 mg of each of its 26 components).

### Example 6

### Study of antidepressant activity of the formulation of this invention in the form of injection

The study of antidepressant activity was conducted with the formulation of the present invention in the form of injection whose preparation was described in Example 1.

The study was performed on sixty-seven (67) women (44-80 years old) suffering from depressive syndrome. They are divided onto two groups by randomization:
(i) a control group (N = 32) received once-α-day dose of 20 mg of sulpiride (**1**) diluted with physiological solution (2.4 mL) by subcutaneous injection during three months (90 days); whereas
(ii) experimental group (N = 35) received once-a-day one ampule (2.4 mL) of the formulation from Example 1 containing 20 mg sulpiride (**1**) and 520 mg of homeopathic modified Krebs solution, by subcutaneous injection during three months (90 days);

The study was followed by the use of Hamilton rating scale for depression (HAMD test). Results are given in Table 2.

### Conclusion

The results obtained in the study represent a clear evidence that the homeopathic modified Krebs solution does enhance antidepressant activity of sulpiride (1).

Literature data show that sulpiride exhibits similar range of efficacy at dosages of at least 150 mg/day. In contrast, our results prove that this dosage can be decreased to only 20 mg/day with preserved high antidepressant activity.

Moreover, beside a profound antidepressant activity, the results from the Hamilton test (HAMD) strongly suggest that the disclosed formulation also has anxiolytic and hypnotic action.

Additionally, drastically reduced (approx. 7x) total dosage (intake) of sulpiride provides a therapy with total absence of unwanted side-effects.

## Claims

1. A therapeutic formulation, consisting of:
(i) sulpiride (1); 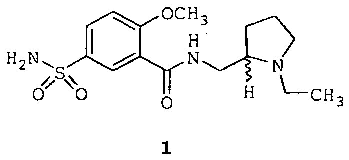
(ii) a homeopathic composition comprising citric acid (D8), *cis-*aconitic acid (D8), α-ketoglutaric acid (D8), succinic acid (D10), fumaric acid (D8), DL-malic acid (D8), sodium diethyloxaloacetate (D6), sodium pyruvate (D8), barium oxalosuccinate (D10), coenzym A (D8), nicotinamide adenine dinucleotide (NAD; D8), adenosine triphosphate (ATP; D10), ascorbic acid (D6), thiamine hydrochloride (D6), sodium riboflavin phosphate (D6), pyridoxine hydrochloride (D6), nicotinamide (D6), cysteine (D6), DL-α-lipoic acid (D6), sulphur (D10), liver of sulphur (D10), magnesium orotate (D6), cerium oxalate (D8), manganese phosphate (D6), the Small Pasque flower extract (*Pulsatilla pratensis L.;* D6), the Red beet extract (*Beta vulgaris ssp. vulgaris var. conditiva,* D4), all of them in equal relative amounts; and
(iii)one or more excipients which yield in desired final dosage form: injection, tablets, capsules, oral solution, and syrup.

2. Formulation according to claim 1, **characterized by** that the excipient is selected from one or more groups consisting of diluents, isotonic salts, emulsifiers, thickeners, fillers, binders, disintegrants, lubricants, preservatives, antioxidants, and stabilizers.

3. Formulation according to claim 2, **characterized by** that the excipient is diluent selected from the group consisting of: purified water, ethanol, glycerol, 1,2-propyleneglycol, 1,3-propyleneglycol, 1,3-butanediol, liquid polyethyleneglycols, liquid polyglycerols, aqueous sorbitol, dimethylsulfoxide, honey, or mixtures of these substances.

4. Formulation according to claim 2, **characterized by** that the excipient is isotonic salt selected from the group consisting of sodium chloride (NaCl), potassium chloride (KCl), magnesium chloride (MgCl₂) and its hydrates, calcium chloride (CaCl₂) and its hydrates, or mixtures of these substances.

5. Formulation according to claim 2, **characterized by** that the excipient is emulsifier selected from the group consisting of: sodium laurylsulphate, sodium lauryl ethyleneglycolsulphate, sodium lauryl diethyleneglycolsulphate, potassium laurylsulphate, potassium lauryl ethyleneglycolsulphate, potassium lauryl diethyleneglycolsulphate; sodium or potassium cocoamphodipropionate; disodium or dipotassium cocoamphodiacetate; polyoxyethylene(10) laurylether, polyoxyethylene(23) laurylether, polyoxyethylene(10) stearylether; polyoxyethylene(23) stearylether; polyoxyethylene(10) oleylether; polyoxyethylene(23) oleylether, other ethoxylates of higher fatty alcohols with H.L.B. value ≥10; polyoxyethylene(10) laurate, polyoxyethylene(23) laurate, polyoxyethylene(10) stearate, polyoxyethylene(23) stearate, polyoxyethylene(10) oleate, polyoxyethylene(23) oleate, other ethoxylates of higher fatty acids with H.L.B. value ≥10; polyglyceryl derivatives of higher fatty alcohols with H.L.B. value ≥10; polyglyceryl derivatives of higher fatty acids with H.L.B. value ≥10; polyoxyethylene sorbitan monolaurate, polyoxyethylene sorbitan monostearate, polyoxyethylene sorbitan monooleate, other sorbitan derivatives with H.L.B. value ≥10; or mixtures of these substances.

6. Formulation according to claim 2, **characterized by** that the excipient is thickener selected from the group consisting of: polyacrylic acid, its co-polymers, or their sodium, or potassium salts; methylcellulose; sodium carboxymethylcellulose; 2-hydroxyethylcellulose; 2-hydroxypropylcellulose; starch; modified starches; polyglycerols; polyethyleneglycols; gelatin; pectin; agar agar; carrageenans; gum arabic; alginic acid; sodium alginate; or mixtures of these substances.

7. Formulation according to claim 2, **characterized by** that the excipient is filler selected from the group consisting of microcrystalline cellulose, lactose monohydrate, calcium hydrogenphosphate, calcium sulfate dihydrate, calcium carbonate, colloidal silicic acid, sorbitol, inulin, starch, modified starches, dextrin, glucose, fructose, basic magnesium carbonate, calcium silicate, saccharose, or mixtures of these substances.

8. Formulation according to claim 2, **characterized by** that the excipient is binder selected from the group consisting of gelatin, lactose monohydrate, sorbitol, saccharose, xylitol, maltitol, starch, modified starches, methylcellulose, 2-hydroxyethylcellulose, 2-hydroxypropylcellulose, 2-hydroxypropyl methylcellulose, sodium carboxymethylcellulose, polyethyleneglycols, polyglycerols, polyvinylpyrrolidone, polyvinylpyrrolidone co-polymers, carrageenans, maltodextrin, or mixtures of these substances.

9. Formulation according to claim 2, **characterized by** that the excipient is disintegrant selected from the group consisting of starch, modified starches, sodium starch glycolate, methylcellulose, sodium carboxymethylcellulose, 2-hydroxyethylcellulose, 2-hydroxypropylcellulose, 2-hydroxypropyl methylcellulose, polyvinylpyrrolidone, polyvinylpyrrolidone co-polymers, or mixtures of these substances.

10. Formulation according to claim 2, **characterized by** that the excipient is lubricant selected from the group consisting of magnesium stearate, calcium stearate, zinc stearate, stearic acid, talc, silicon dioxide, or mixtures of these substances.

11. Formulation according to claim 2, **characterized by** that the excipient is preservative selected from the group consisting of methyl 4-hydroxybenzoate, ethyl 4-hydroxybenzoate, propyl 4-hydroxybenzoate, butyl 4-hydroxybenzoate, sorbic acid, potassium sorbate, benzoic acid, sodium benzoate, 2-phenoxyethanol, 4-chloro-m-cresol, thymol, eugenol, or mixtures of these substances.

12. Formulation according to claim 2, **characterized by** that the excipient is antioxidant selected from the group consisting of 2,6-di-*terc*-butyl-4-hydroxytoluene (BHT), *terc-*butylhydroxyanisole (BHA), tocopherol, tocopheryl acetate, ascorbic acid, or mixtures of these substances.

13. Formulation according to claim 2, **characterized by** that the excipient is stabilizer selected from the group consisting of disodium ethylenediamine tetraacetate (Na₂EDTA·2H₂O), disodium N-(2-hydroxyethyl)ethylenediamine triacetate [Na₂H(HEDTA)], disodium diethylenetriamine pentaacetate [Na₂H₃(DTPA)], disodium citrate [Na₂C (OH) (COOH) (CH₂COO)₂], or mixtures of these substances.

14. Formulation according to claims 1-2 with the excipient selected according to one or more claims 3-13 **wherein**:
(i) sulpiride is present in the amount of 0.1-25% w/w;
(ii) a homeopathic composition in the amount of 10-75% w/w; and
(iii) one or more excipients to yield 100% w/w of the formulation.

15. Formulation according to any of the preceding claims for use as a therapeutic agent for treatment of depressive syndrome, anxiety, and as a therapeutic hypnotic agent.

## Patentansprüche

1. Eine therapeutische Formulierung, bestehend aus:
(i) Sulpirid (1); 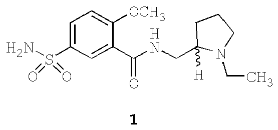
(ii) eine homöopathische Zusammensetzung, umfassend Zitronensäure (D8), cis-Aconitsäure (D8), α-Ketoglutarsäure (D8), Bernsteinsäure (D10), Fumarsäure (D8), DL-Äpfelsäure (D8), Natrium Diethyloxaloacetat (D6), Natriumpyruvat (D8), Bariumoxalsuccinat (D10), Coenzym A (D8), Nicotinamid-Adenin-Dinukleotid (NAD; D8), Adenosintriphosphat (ATP; D10), Ascorbinsäure (D6), Thiaminhydrochlorid (D6), Riboflavin-5'-(natriumhydrogenphosphat) (D6), Pyridoxinhydrochlorid (D6), Nicotinamid (D6), Cystein (D6), DL-α-Liponsäure (D6), Schwefel (D10), Schwefelleber (D10), Magnesium-Orotat (D6), Cer(III)-oxalat (D8), Manganphosphat (D6), Extrakt der Wiesenkuhschelle (*Pulsatilla pratensis* L.; D6), Extrakt der Roten Bete (Beta *vulgaris ssp. vulgaris var. conditiva*, D4), alle in gleichen relativen Mengen; und
(iii) ein oder mehrere Excipientien, welche in der gewünschten endgültigen Dosierform ergeben: Injektion, Tabletten, Kapseln, orale Lösung, und Sirup.

2. Formulierung nach Anspruch 1, **dadurch gekennzeichnet, dass** das Excipiens ausgewählt ist aus einer oder mehrerer Gruppen, bestehend aus Diluentien, isotonischen Salzen, Emulgatoren, Verdickungsmitteln, Füllstoffen, Bindemitteln, Sprengmitteln, Gleitmitteln, Konservierungsstoffen, Antioxidantien, und Stabilisatoren.

3. Formulierung nach Anspruch 2, **dadurch gekennzeichnet, dass** das Excipiens ein Diluens ist, ausgewählt aus der Gruppe, bestehend aus gereinigtem Wasser, Ethanol, Glycerin, 1,2-Propylenglycol, 1,3-Propylenglycol, 1,3-Butandiol, flüssigem Polyethylenglycol, flüssigem Polyglycerin, wässrigem Sorbitol, Dimethylsulfoxid, Honig, oder Mischungen dieser Substanzen.

4. Formulierung nach Anspruch 2, **dadurch gekennzeichnet, dass** das Excipiens ein isotonisches Salz ist, ausgewählt aus der Gruppe, bestehend aus Natriumchlorid (NaCl), Kaliumchlorid (KCl), Magnesiumchlorid (MgCl₂) und seiner Hydrate, Kalciumchlorid (CaCl₂) und seiner Hydrate, oder Mischungen dieser Substanzen.

5. Formulierung nach Anspruch 2, **dadurch gekennzeichnet, dass** das Excipiens ein Emulgator ist, ausgewählt aus der Gruppe, bestehend aus: Natriumlaurylsulfat, Natrium-laurylethylenglycolsulfat, Natrium-lauryl-diethylenglycolsulfat, Kaliumlaurylsulfat, Kalium-lauryl-ethylenglycolsulfat, Kalium-lauryl-diethylenglycolsulfat; Natrium oder Kalium Cocoamphodipropionat; Dinatrium oder Dikalium Cocoamphodiacetat; Polyoxyethylen(10) laurylether, Polyoxyethylen(23) laurylether, Polyoxyethylen(10) stearylether; Polyoxyethylen(23) stearylether; Polyoxyethylen(10) oleylether; Polyoxyethylen(23) oleylether, anderen Ethoxylaten höherer Fettalkohole mit H.L.B. Wert ≥10; Polyoxyethylen(10)-Laurat, Polyoxyethylen(23)-Laurat, Polyoxyethylen(10)-Stearat, Polyoxyethylen(23)-Stearat, Polyoxyethylen(10)-Oleat, Polyoxyethylen(23)-Oleat, anderen Ethoxylaten höherer Fettsäuren mit H.L.B. Wert ≥10; Polyglycerolderivaten höherer Fettalkohole mit H.L.B. Wert ≥10; Polyglycerolderivaten höherer Fettsäuren mit H.L.B. Wert ≥10; Polyoxyethylen-sorbitan-monolaurat, Polyoxyethylen- sorbitanmonostearat, Polyoxyethylen-sorbitan-monooleat, anderen Sorbitanderivaten mit H.L.B. Wert ≥10; oder Mischungen dieser Substanzen.

6. Formulierung nach Anspruch 2, **dadurch gekennzeichnet, dass** das Excipiens ein Verdickungsmittel ist, ausgewählt aus der Gruppe, bestehend aus: Polyacrylsäure, ihrer Copolymeren, oder ihrer Natrium-, oder Kaliumsalze; Methylcellulose; NatriumCarboxymethylcellulose; 2-Hydroxyethylcellulose; 2-Hydroxypropylcellulose; Stärke; modifizierter Stärke; Polyglycerin; Polyethylenglycol; Gelatine; Pektin; Agar Agar; Carrageen; Gummi arabicum; Alginsäure; Natrium-Alginat; oder Mischungen dieser Substanzen.

7. Formulierung nach Anspruch 2, **dadurch gekennzeichnet, dass** das Excipiens ein Füllstoff ist, ausgewählt aus der Gruppe, bestehend aus Mikrokristalliner Cellulose, Lactose-Monohydrat, Calciumhydrogenphosphat, Calciumsulfat Dihydrat, Calciumcarbonat, kolloidaler Kieselsäure, Sorbitol, Inulin, Stärke, modifizierter Stärke, Dextrin, Glucose, Fructose, basischem Magnesiumkarbonat, Calciumsilicat, Saccharose, oder Mischungen dieser Substanzen.

8. Formulierung nach Anspruch 2, **dadurch gekennzeichnet, dass** das Excipiens ein Bindemittel ist, ausgewählt aus der Gruppe, bestehend aus Gelatine, Lactose-Monohydrat, Sorbitol, Saccharose, Xylit, Maltit, Stärke, modifizierter Stärke, Methylcellulose, 2-Hydroxyethylcellulose, 2-Hydroxypropylcellulose, 2-Hydroxypropylmethylcellulose, Natrium Carboxymethylcellulose, Polyethylenglycol, Polyglycerin, Polyvinylpyrrolidon, Polyvinylpyrrolidon Copolymer, Carrageen, Maltodextrin, oder Mischungen dieser Substanzen.

9. Formulierung nach Anspruch 2, **dadurch gekennzeichnet, dass** das Excipiens ein Sprengmittel ist, ausgewählt aus der Gruppe, bestehend aus Stärke, modifizierter Stärke, Natrium-Stärke-Glycolat, Methylcellulose, Natrium Carboxymethylcellulose, 2-Hydroxyethylcellulose, 2-Hydroxypropylcellulose, 2-Hydroxypropylmethylcellulose, Polyvinylpyrrolidon, Polyvinylpyrrolidon Copolymer, oder Mischungen dieser Substanzen.

10. Formulierung nach Anspruch 2, **dadurch gekennzeichnet, dass** das Excipiens ein Gleitmittel ist, ausgewählt aus der Gruppe, bestehend aus Magnesiumstearat, Calciumstearat, Zinkstearate, Stearinsäure, Talk, Silikondioxid, oder Mischungen dieser Substanzen.

11. Formulierung nach Anspruch 2, **dadurch gekennzeichnet, dass** das Excipiens ein Konservierungsstoff ist, ausgewählt aus der Gruppe, bestehend aus Methyl 4-Hydroxybenzoat, Ethyl 4-Hydroxybenzoat, Propyl 4- Hydroxybenzoat, Butyl 4-Hydroxybenzoat, Sorbinsäure, Kaliumsorbat, Benzoesäure, Natriumbenzoat, 2-Phenoxyethanol, 4-Chlor-m-kresol, Thymol, Eugenol, oder Mischungen dieser Substanzen.

12. Formulierung nach Anspruch 2, **dadurch gekennzeichnet, dass** das Excipiens ein Antioxidan ist, ausgewählt aus der Gruppe, bestehend aus 2,6-Di-tert-butyl-4-hydroxytoluol (BHT), tert-Butylhydroxyanisol (BHA), Tocopherol, Tocopherylacetat, Ascorbinsäure, oder Mischungen dieser Substanzen.

13. Formulierung nach Anspruch 2, **dadurch gekennzeichnet, dass** das Excipiens ein Stabilisator ist, ausgewählt aus der Gruppe, bestehend aus Dinatrium-ethylendiamin-tetraacetat (Na₂EDTA·2H₂O), Dinatrium N-(2-hydroxyethyl)ethylendiamin triacetat [Na₂H(HEDTA)], Dinatrium diethylentriamin pentaacetat [Na₂H3(DTPA)], Dinatriumcitrat [Na₂C(OH) (COOH) (CH₂COO)₂], oder Mischungen dieser Substanzen.

14. Formulierung nach den Ansprüchen 1-2 mit dem Excipiens, ausgewählt nach einem oder mehrerer Ansprüche 3-13, wobei:
(i) Sulpirid in einer Menge von 0.1-25% Gew./Gew. zugegen ist;
(ii) eine homöopathische Zusammensetzung in einer Menge von 10-75% Gew./Gew.; und
(iii) ein oder mehrere Excipienten, um 100% Gew./Gew. der Formulierung zu erhalten.

15. Formulierung nach einem der vorhergehenden Ansprüche zur Verwendung als therapeutisches Mittel zur Behandlung des depressiven Syndroms, Angstgefühlen, und als therapeutisches Hypnotikum.

## Revendications

1. Une formulation thérapeutique, comprenant:
(i) le sulpiride (1); 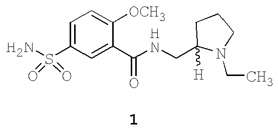
(ii) une composition homéopathique comprenant l'acide citrique (D8), l'acide cis-aconitique (D8), l'acide α-cétoglutarique (D8), l'acide succinique (D10), l'acide fumarique (D8), l'acide DL-malique (D8), le diéthylomaloacétate de sodium (D6), le pyruvate de sodium (D8), l'oxalosuccinate de baryum (D10), le coenzyme A (D8), le nicotinamide-adénine-dinucléotide (NAD; D8), l'adénosine-triphosphate (ATP ; D10), l'acide ascorbique (D6), l'hydrochlorure de thiamine (D6), le phosphate sodique de riboflavine (D6), l'hydrochlorure de pyridoxine (D6), la nicotinamide (D6), la cystéine (D6), l'acide DL-α-lipoïque (D6), le soufre (D10), le foie du soufre (D10), l'orotate de magnésium (D6), l'oxalate de cérium (D8), le phosphate de manganèse (D6), l'extrait de fleur de Pulsatille des prés (*Pulsatilla pratensis* L.; D6), l'extrait de Betterave rouge (*Beta vulgaris ssp. vulgaris var. conditiva,* D4), tous dans la même quantité relative; et
(iii) un ou plusieurs excipients qui contribuent à la forme posologique finale désirée: injection, comprimés, capsules, solution orale, et sirop.

2. Une formulation selon la revendication 1, **caractérisée en ce que** l'excipient est sélectionné parmi un ou plusieurs groupes consistant des diluants, des sels isotoniques, des émulsifiants, des épaississants, des agents de charge, des liants, des désintégrants, des lubrifiants, des conservateurs, des antioxydants, et des agents stabilisants.

3. Une formulation selon la revendication 2, **caractérisée en ce que** l'excipient est diluant sélectionné parmi le groupe consistant de l'eau purifiée, l'éthanol, le glycérol, le 1,2-propylène-glycol, le 1,3-propylène-glycol, le butane-1,3-diol, les polyéthylène-glycols liquides, les polyglycérols liquides, le sorbitol aqueux, le diméthylsulfoxide, le miel, ou les mélanges desdites substances.

4. Une formulation selon la revendication 2, **caractérisée en ce que** l'excipient est sel isotonique sélectionné parmi le groupe consistant du chlorure de sodium (NaCl), le chlorure de potassium (KCl), le chlorure de magnésium (MgCl₂) et ses hydrates, le chlorure de calcium (CaCl₂) et ses hydrates, ou les mélanges desdites substances.

5. Une formulation selon la revendication 2, **caractérisée en ce que** l'excipient est émulsifiant sélectionné parmi le groupe consistant du laurylsulfate de sodium, le lauryl-éthylène-glycol-sulfate de sodium, le lauryl-diéthylène-glycol-sulfate de sodium, le laurylsulfate de potassium, le lauryl-éthylène-glycol-sulfate de potassium, le lauryl-diéthylène-glycol-sulfate de potassium; le cocoamphodipropionate de sodium ou potassium; le cocoamphodiacétate de disodium ou dipotassium; le polyomyéthylène(10)-lauryléther, le polyoxyéthylène(23)-lauryléther, le polyomyéthylène(10)-stéaryléther; le polyomyéthylène(23)-stéaryléther; le polyoxyéthylène(10)-oleyléther; le polyomyéthylène(23)-oleyléther, autres éthoxylates des alcools gras supérieurs avec une valeur de HLB ≥10; le polyoxyéthylène(10)-laurate, le polyoxyéthylène(23)-laurate, le polyomyéthylène(10)-stéarate, le polyoxyéthylène(23)-stéarate, le polyoxyéthylène(10)-oléate, le polyoxyéthylène(23)-oléate, autres éthoxylates des acides gras supérieurs avec une valeur de HLB >10; dérivés polyglycériques des alcools gras supérieurs avec une valeur de HLB ≥10; dérivés polyglycériques des acides gras supérieurs avec une valeur de HLB ≥10; le monolaurate de polyoxyéthylène-sorbitane, le monostéarate de polyoxyéthylène-sorbitane, le monooléate de polyoxyéthylène-sorbitane, autres dérivés de sorbitane avec une valeur de HLB ≥10; ou les mélanges desdites substances.

6. Une formulation selon la revendication 2, **caractérisée en ce que** l'excipient est épaississant sélectionné parmi le groupe consistant de l'acide polyacrylique, ces copolymères, ou leurs sels sodiques ou potassiques; la méthylcellulose; la carboxyméthylcellulose sodique; la 2-hydroxyéthylcellulose; la 2-hydroxypropylcellulose; l'amidon; les amidons modifiés; les polyglycérols; les polyéthylèneglycols; la gélatine; la pectine; l'agar-agar; le carraghénane; la gomme arabique; l'acide alginique; l'alginate de sodium; ou les mélanges desdites substances.

7. Une formulation selon la revendication 2, **caractérisée en ce que** l'excipient est agent de charge sélectionné parmi le groupe consistant de la cellulose microcristalline, le lactose monohydraté, l'hydrogénophosphate de calcium, le sulfate de calcium dihydraté, le carbonate de calcium, l'acide silicique colloïdal, le sorbitol, l'inuline, l'amidon, les amidons modifiés, la dextrine, le glucose, le fructose, le carbonate de magnésium basique, le silicate de calcium, le saccharose, ou les mélanges desdites substances.

8. Une formulation selon la revendication 2, **caractérisée en ce que** l'excipient est liant sélectionné parmi le groupe consistant de la gélatine, le lactose monohydraté, le sorbitol, le saccharose, le xylitol, le maltitol, l'amidon, les amidons modifiés, la méthylcellulose, la 2-hydroxyéthylcellulose, la 2-hydroxypropylcellulose, la 2-hydroxypropylméthylcellulose, la carboxyméthylcellulose sodique, les polyéthylèneglycols, les polyglycérols, la polyvinylpyrrolidone, les copolymères de polyvinylpyrrolidone, le carraghénane, la maltodextrine, ou les mélanges desdites substances.

9. Une formulation selon la revendication 2, **caractérisée en ce que** l'excipient est désintégrant sélectionné parmi le groupe consistant de l'amidon, les amidons modifiés, le glycolate sodique d'amidon, la méthylcellulose, la carboxyméthylcellulose sodique, la 2-hydroxyéthylcellulose, la 2-hydroxypropylcellulose, la 2-hydroxypropylméthylcellulose, la polyvinylpyrrolidone, les copolymères de polyvinylpyrrolidone, ou les mélanges desdites substances.

10. Une formulation selon la revendication 2, **caractérisée en ce que** l'excipient est lubrifiant sélectionné parmi le groupe consistant du stéarate de magnésium, le stéarate de calcium, le stéarate de zinc, l'acide stéarique, le talc, le dioxyde de silicium, ou les mélanges desdites substances.

11. Une formulation selon la revendication 2, **caractérisée en ce que** l'excipient est conservateur sélectionné parmi le groupe consistant du méthyl-4-hydroxybenzoate, l'éthyl-4-hydroxybenzoate, le propyl-4-hydroxybenzoate, le butyl-4-hydroxybenzoate, l'acide sorbique, le sorbate de potassium, l'acide benzoïque, le benzoate de sodium, le 2-phénoxyéthanol, le 4-chloro-M-cresol, le thymol, l'eugénol, ou les mélanges desdites substances.

12. Une formulation selon la revendication 2, **caractérisée en ce que** l'excipient est antioxydant sélectionné parmi le groupe consistant du 2,6-di-*tert*-butyl-4-hydroxytoluène (BHT), le *tert*-butylhydroxyanisole (BHA), le tocophérol, l'acétate de tocophéryl, l'acide ascorbique, ou les mélanges desdites substances.

13. Une formulation selon la revendication 2, **caractérisée en ce que** l'excipient est agent stabilisant sélectionné parmi le groupe consistant de l'éthylène-diamine-tétraacétate de disodium (Na₂EDTA•2H₂O), le N-(2-hydroxyéthyl)-éthylène-diamine-triacétate de disodium [Na₂H(HEDTA)], le diéthylène-triamine-pentaacétate de disodium [Na₂H₃(DTPA)], le citrate de disodium [Na₂C(OH)(COOH) (CH₂COO)₂], ou les mélanges desdites substances.

14. Une formulation selon les revendications 1-2 avec l'excipient sélectionné selon une ou plusieurs revendications 3-13 où:
(i) le sulpiride est présent dans la quantité de 0,1-25% masse/masse;
(ii) une composition homéopathique dans la quantité de 10-75% masse/masse; et
(iii) un ou plusieurs excipients qui contribuent à atteindre les 100% masse/masse de la formulation.

15. Une formulation selon une quelconque des revendications précédentes pour l'usage comme agent thérapeutique pour le traitement du syndrome dépressif, de l'anxiété, et comme agent thérapeutique hypnotique.
